# EUROPÄISCHE PATENTSCHRIFT

(11) **EP 2 747 673 B1**
(45) Veröffentlichungstag und Bekanntmachung des Hinweises auf die Patenterteilung: **01.03.2017**
(21) Anmeldenummer: 12758428.2
(22) Anmeldetag: 24.08.2012
(51) Int. Cl.: A61B 17/15

(54) **SCHNEIDFÜHRUNG ZUM ERZEUGEN EINER AUSSENKONTUR FÜR EINE GELENKENDOPROTHESE**
CUTTING GUIDE FOR CREATING AN EXTERNAL CONTOUR FOR AN ARTICULATED ENDOPROSTHETIC
GUIDAGE DE COUPE POUR PRODUIRE UN CONTOUR EXTERNE POUR UNE ENDOPROTHÈSE ARTICULÉE

(30) Priorität: 25.08.2011 EP 11178804; 26.08.2011 US 201161527885 P
(43) Veröffentlichungstag der Anmeldung: 02.07.2014
(73) Patentinhaber: Waldemar Link GmbH & Co. KG, 22339 Hamburg (DE)
(72) Erfinder: DMUSCHEWSKY, Klaus, 22397 Hamburg (DE)
(74) Vertreter: Hoffmann Eitle
(86) Internationale Anmeldenummer: PCT/EP2012/066517
(87) Internationale Veröffentlichungsnummer: WO 2013/026926

(56) Entgegenhaltungen:
- WO-A1-96/07361
- WO-A2-2010/085538
- DE-A1- 10 309 493
- US-A1- 2003 171 757
- US-A1- 2004 153 066

## Beschreibung

### TECHNISCHES GEBIET

Die Erfindung betrifft eine Schneidführung zum Erzeugen einer Außenkontur an einem Knochen zum Anbringen einer Gelenkendoprothese, insbesondere einer Femurkomponente einer Knieendoprothese.

### STAND DER TECHNIK

Arthrose, d.h. der übermäßige Gelenksverschleiß, bedeutet für Betroffene eine signifikante Einschränkung der Lebensqualität. Dieses mit starken Schmerzen verbundene Krankheitsbild tritt allerdings nicht nur im höheren Alter auf, sondern immer stärker auch bei jüngeren Patienten, beispielsweise verursacht durch Übergewicht und als Folge von Sportunfällen. Trotz einer Vielzahl entwickelter Behandlungsmethoden ermöglicht letztendlich häufig nur ein künstliches Gelenk einen Rückgewinn schmerzfreier Bewegungsfreiheit.

Allerdings sind auch diese künstlichen Gelenkimplantate von Verschleiß betroffen, was dazu führt, dass insbesondere bei jüngeren Patienten das künstliche Gelenk durch ein neues ersetzt werden muss. Eine solche Revision kann unter Umständen sogar mehrfach notwendig sein. Jede Revision setzt jedoch das Vorhandensein eines ausreichenden Knochenstocks voraus, und es ist beim Gelenkersatz wichtig, dass dieser die vorhandenen Knochen- und Bänderstrukturen weitgehend erhält.

Zum Erreichen dieses Ziels wurde der so genannte Oberflächenersatz entwickelt. Der Oberflächenersatz ersetzt lediglich die Gelenkoberfläche und erfordert nur eine relativ geringfügige Resektion des Knochens. Weiterhin kann bei dieser Technik der das Gelenk umschließende Bänderapparat weitestgehend erhalten werden. Ein solcher Oberflächenersatz kann entweder einseitig oder zweiseitig erfolgen. Dabei kann auf der jeweiligen Gelenkseite der Ersatz über die gesamten Gelenkflächen, z.B. beim Hüftgelenk, oder auch nur abschnittsweise erfolgen, z.B. an den lateralen oder medialen Femurkondylen und Tibiakopf beim Kniegelenk.

Erfolgt der Gelenk- oder Oberflächenersatz bei der so genannten Hemiarthroplastie nur einseitig, hat nach erfolgter Implantation die künstliche Gelenkoberfläche den Gelenkknorpel als Gleitpartner. Bei dem hauptsächlich durchgeführten zweiseitigen Ersatz (Totalersatz) werden beide einander gegenüberliegende Gelenkoberflächen durch künstliche Gelenkoberflächen von Gelenkprothesen ersetzt, die zumeist aus Metall, Kunststoff und/oder Keramik bestehen. Besonders häufig wird ein solcher Oberflächenersatz im Kniegelenk eingesetzt. Dieser erfolgt sowohl uni- als auch bikondylär. Allerdings ist der Oberflächenersatz auch durchaus bei anderen Gelenken, wie beispielsweise dem Hüftgelenk, dem Schultergelenk oder dem Sprunggelenk einsetzbar.

Eine Voraussetzung für eine optimale Funktionalität des Gelenkersatzes ist seine gute Positionierung und Ausrichtung, um nach der Operation auftretende Fehlstellungen wie zum Beispiel X-Beine oder O-Beine zu vermeiden oder auch durch eine gegenüber dem echten Gelenk unterschiedliche Positionierung der Gelenkendoprothese zu korrigieren. Selbstverständlich ist es genauso wichtig, das Implantat entsprechend korrekt zu positionieren und auszurichten, um eine einwandfreie Kinematik des Gelenks sicherzustellen. Dies ist vor allem dann eine Herausforderung für den Operateur, wenn zum Beispiel bei einem Kniegelenk lediglich die mediale oder laterale Seite des Gelenks durch eine Gelenkendoprothese ersetzt wird. Die einwandfreie Positionierung und Ausrichtung ist genauso wichtig, wenn ein Implantat zumindest auf einer Seite aus mehreren zusammenwirkenden Gelenkflächen besteht, die jedoch getrennt voneinander implantiert werden. Bei dem Kniegelenk haben diese mehrteiligen Implantate insbesondere den Vorteil, dass sie erstens den Knochenstock und zweitens den Bänderapparat des Knies schonen. Im Gegensatz dazu resultiert ein gekoppelter Gelenkersatz in der Regel in dem Verlust des vorderen und hinteren Kreuzbands im Knie.

Um beim Implantieren bzw. Anbringen eines Gelenkersatzes eine möglichst exakte und schnelle Positionierung sicherzustellen, können Schneidführungen eingesetzt werden. Diese Führungen werden während der Operation jeweils an dem Ende des Knochens, wo die Gelenkendoprothese implantiert werden soll, positioniert und ausgerichtet. Die Resektion des Knochens findet anschließend beispielsweise mit Hilfe von Sägen, Raspeln oder Fräsen statt, deren Schnittrichtung von den Führungen vorgegeben ist. Auf der durch die Resektion erzeugten knöchernen Außenkontur wird das Implantat schließlich verankert, indem es zementiert oder zementfrei eingepresst wird. Nach anfänglicher Presspassung wächst der Knochen im Laufe der Zeit schließlich in das Implantat ein.

Eine Schneidführung gemäß des Oberbegriffs des unabhängigen Anspruchs 1 der vorliegenden Erfindung zur Resektion von Knochen für die anschließende Implantation einer Gelenkendoprothese wird in der US 2003/0171757 A1 offenbart. Insbesondere werden in der genannten Patentanmeldung ein Verfahren und eine Vorrichtung zum Ausführen einer minimalinvasiven totalendoprothetischen Versorgung eines Kniegelenks offenbart, die Komponenten mit Führungsflächen und Schlitzen zum Festlegen eines Pfads für eine Säge einschließt. Das Instrumentarium ermöglicht die Resektion des proximalen Endes der Tibia und des distalen Endes des Femurs, und zwar sowohl medial als auch lateral mit minimalem Einschnitt in Weichteilgewebe.

Das Dokument US2004153066 A1 offenbart eine Schneidführung gemäß dem Oberbegriff von Anspruch 1.

Bedingt durch die relativ komplexe Geometrie der zu bearbeitenden und der zu erzeugenden Außenkonturen ist während der Erzeugung der Außenkontur häufig ein mehrfaches Umsetzen oder oft auch die Verwendung mehrerer Schneidführungen notwendig. Dies zieht jedoch vor allem zwei Nachteile mit sich. Erstens ist die erreichbare Genauigkeit bezüglich der zu erzeugenden Außenkontur beeinträchtigt, u.a. weil die nach Umsetzen der Führung erzeugten Außenkonturen nicht mit den vor dem Umsetzen erzeugten fluchten. Zweitens erfordert das Umsetzen und/oder Wechseln der Schneidführungen und das erneute Positionieren und Ausrichten zusätzliche Zeit während der Operation. Dies führt wiederum einerseits zu einem erhöhten Risiko für den Patienten, vor allem durch die länger notwendige Narkose sowie andererseits zu einer höheren Infektionsgefahr durch die längere Exposition der Wunde.

### DARSTELLUNG DER ERFINDUNG

Aufgabe der vorliegenden Erfindung war es somit, eine Schneidführung bereitzustellen, die auf möglichst einfache Art und Weise so positioniert und ausgerichtet werden kann, dass ein Umsetzen möglichst vermieden wird. Gleichzeitig soll die Schneidführung möglichst einen gewebeschonenden Eingriff ermöglichen.

Diese Aufgabe wird mit einer Schneidführung und einem Verfahren gelöst, wie sie in den unabhängigen Ansprüchen angegeben sind. Weiterhin definieren die abhängigen Ansprüche bevorzugte Ausführungsformen und Weiterbildungen der Erfindung.

In einer ersten Ausführungsform der vorliegenden Erfindung wird eine Schneidführung zum Erzeugen einer Außenkontur an einem Knochen, wobei an der Außenkontur eine Gelenkendoprothese (70), insbesondere eine Femurkomponente einer Knieendoprothese anbringbar ist, bereitgestellt. Die Schneidführung weist eine sagittale Führungsplatte auf, die zu dem Knochen ausrichtbar ist, und mindestens zwei sagittale Führungsschlitze zum Führen eines knochenschneidenden Werkzeugs, wobei die sagittalen Führungsschlitze durch die sagittale Führungsplatte hindurchgehen und winklig zueinander angeordnet sind. Im Falle eines Kniegelenks ist die sagittale Führungsplatte so am Knochen ausrichtbar, dass sie sich im Wesentlichen in der Sagittalebene des Körpers beziehungsweise parallel hierzu befindet. In diesem Fall durchbrechen die sagittalen Führungsschlitze die Sagittalebene des Körpers.

Die Führungsschlitze sind entsprechend des knochenschneidenden Werkzeugs ausgeführt. Vorzugsweise werden als knochenschneidende Werkzeuge bei der Verwendung der erfindungsgemäßen Schneidführung Sägen, Bohrer, Raspeln und/oder Fräsen eingesetzt. Die Führungsschlitze weisen dann Abmessungen auf, die ein spielfreies Einführen und Bewegen des jeweiligen knochenschneidenden Werkzeugs ermöglichen. Sie verlaufen durch das Material der vertikalen Platten, wobei vorzugsweise zumindest die Längsseiten ihres Querschnitts zueinander parallel sind. Auch wenn der Querschnitt der Führungsschlitze bevorzugt konstant ist, ist es auch möglich, dass sich der Schlitz in seiner Durchgangsrichtung verengt oder aufweitet. Anders gesagt ist bei einem derartig ausgebildeten Führungsschlitz seine Öffnung auf der Werkzeugseintrittsseite dann größer als auf der Werkzeugsaustrittsseite oder umgekehrt.

Die sagittale Führungsplatte weist zumindest auf der dem Gelenk beziehungsweise dem Knochen zugewandten Seite eine im Wesentlichen sagittal verlaufende Seitenfläche auf. Mit anderen Worten befindet sich die sagittal verlaufende Seitenfläche an der Austrittseite der Führungsschlitze, dass heißt in der Richtung in der sich die Führungsschlitze der sagittalen Führungsplatte durch die sagittale Führungsplatte erstrecken. An dieser Seitenfläche sind die sagittalen Führungsschlitze winklig zueinander angeordnet. Hierbei ist anzumerken, dass bei mehr als zwei sagittalen Führungsschlitzen die von den Längsrichtungen benachbarter Führungsschlitze eingeschlossenen Winkel nicht gleich sein müssen, sondern vielmehr in Abhängigkeit von der zu erzeugenden Außenkontur auch unterschiedliche Winkel eingeschlossen werden können. Dabei ist die Außenkontur wiederum von der Innenkontur der Gelenkendoprothese, vorzugsweise des Gelenkoberflächenersatzes, vorgegeben.

Besonders geeignet ist die vorliegende Schneidführung zum Erzeugen von im Wesentlichen facettenartigen Konturen. Dementsprechend sind die Führungsschlitze bevorzugt so angeordnet, dass die durch sie erzeugte Außenkontur am Knochenende aus facettenartig angeordneten, aneinander angrenzenden Flächen besteht. Wenn man zur Lagebezeichnung benachbarter Facetten bzw. Führungsschlitze ein kartesisches Koordinatensystem betrachtet, in dessen einer Ebene, also z.B. der Y-Z Ebene die erste Facette bzw. der erste Führungsschlitz liegt, ist die bzw. der nächste relativ zum ersten um mindestens eine Achse verdreht, z.B. um die Z- und/oder um die Y-Achse.

Es ist vorteilhaft, in der sagittalen Führungsplatte mehr als zwei Führungsschlitze vorzusehen, bevorzugt zwischen vier und zehn und besonders bevorzugt zwischen sechs und acht Führungsschlitze. Der Grund hierfür ist, dass eine höhere Anzahl an Führungsschlitzen die Anzahl der Facetten erhöht und das Erzeugen einer stetigeren Außenkontur ermöglicht, die besser an die anatomische Gelenkoberfläche angenähert ist. Kurz gesagt folgt die so durch die Führungsschlitze erzeugte Schnittlinie unter der Gelenkoberfläche im Wesentlichen ihrer Außenkontur. Dabei ist die Schnittlinie durch die strukturelle Anordnung der Führungsschlitze polygonförmig. Somit wird eine idealerweise bogenförmige Schnittlinie mit einer Vielzahl von tangential ausgerichteten Schnitten angenähert. Je vieleckiger das Polygon bzw. je mehr Führungsschlitze vorhanden sind, desto besser folgt somit die unterhalb der Gelenkoberfläche angeordnete Schnittlinie der Außenkontur der Gelenkoberfläche. Daraus ergibt sich der Vorteil, dass bei der Erzeugung der Außenkontur der Verlust an Knochensubstanz minimiert wird. Die Maximalanzahl an Führungsschlitzen ergibt sich aus der trotz der Schlitze zu gewährleistenden Stabilität der Führung und Handhabbarkeit durch den Chirurgen.

Die in die sagittale Führungsplatte hineingehenden Führungsschlitze sind dabei um im Wesentlichen parallel zur Schlitzbreite liegenden Achse in einem Winkel von 40-80°, bevorzugt 50-70° und besonders bevorzugt 55-65° geneigt. Auch wenn die Neigungsachse bevorzugt parallel zur Schlitzbreite verläuft, so kann sie auch zur Längsrichtung der Schlitzöffnung leicht geneigt sein um Facetten zu erzeugen, die in zwei Winkeln zueinander verdreht sind. Die Neigung verläuft, betrachtet von der Außenseite zur Gelenkseite der sagittalen Platte, bevorzugt in Richtung der Bogeninnenseite. Die auf der sagittalen Platte fächerartig zueinander ausgerichteten Schlitze stehen in einem Winkel von 10-30°, bevorzugt 12-28°, besonders bevorzugt 15-25° zueinander. Anders ausgedrückt verlaufen die Führungsschlitze mit ihrer Öffnungslängsrichtung von der zu erzeugenden bogenförmigen Schnittlinie tangential strahlenförmig von der bogenförmigen Schnittlinie weg, ohne dass dabei die Strahlen einen gemeinsamen Schnittpunkt aufweisen. Die Winkel zwischen den einzelnen Führungsschlitzen werden dabei im Durchschnitt kleiner je mehr Facetten zur Annährung an die bogenförmige Ideallinie parallel zur anatomischen Gelenkoberfläche verwendet werden. Allerdings können die einzelnen Winkel durchaus voneinander abweichen.

Durch die oben beschriebene Anordnung der Führungsschlitze in der sagittalen Führungsplatte wird die gewünschte Außenkontur an dem zu ersetzenden Gelenk ohne ein Umsetzen der Schneidführung oder das Verwenden zusätzlicher Schneidführungen erzeugt. Dies hat zum einen den Vorteil, dass die Operationszeit verkürzt wird und andererseits die Genauigkeit der Außenkontur reproduzierbar erhöht wird.

In einer weiteren bevorzugten Ausführungsform sind die sagittalen Führungsschlitze so innerhalb der sagittalen Führungsplatte angeordnet, dass sie sich im Wesentlichen nicht gegenseitig überschneiden. Somit bildet jeder Führungsschlitz eine in sich abgeschlossene Öffnung. Eine in sich abgeschlossene Öffnung im Sinne der vorliegenden Erfindung liegt dann vor, wenn sich die Öffnung zumindest an ihrer Werkzeugeintrittsseite oder ihrer Werkzeugaustrittsseite nicht mit der Öffnung eines anderen Führungsschlitzes überschneidet. Um also das knochenschneidende Werkzeug von einem Führungsschlitz zum nächsten zu führen, muss das Werkzeug komplett aus einem Führungsschlitz herausgenommen werden, um anschließend in einen anderen wieder eingeführt werden zu können. Um trotz der sich nicht überschneidenden Führungsschlitze die durchgehende facettenförmige Außenkontur zu erzeugen, muss der Schlitz entsprechend lang ausgeführt sein, damit das knochenschneidende Werkzeug die von der sagittalen Führungsplatte verdeckten Knochenabschnitte beispielsweise durch schräges Einführen durchtrennen kann.

Voneinander räumlich getrennte Führungsschlitze haben den Vorteil, dass das knochenschneidende Werkzeug sich nicht verklemmen kann, wie es sonst aufgrund des Übergangs zwischen zwei Führungsschlitzen der Fall sein könnte. Verklemmt sich nämlich das Werkzeug, wirkt seine Bewegung direkt auf die Schneidführung, so dass eine korrekte Positionierung und Ausrichtung der Schneidführung nicht mehr gewährleistet werden kann.

Bei einer weiteren Ausführungsform der vorliegenden Erfindung sind die Führungsschlitze im Wesentlichen auf einer Bogenlinie verteilt angeordnet. Dabei sind die Führungsschlitze vorzugsweise nicht entlang der Bogenlinie ausgerichtet, sondern stattdessen vorzugsweise so, dass sie mit ihrer Öffnungslängsrichtung winklig zu der Bogenlinie stehen und relativ zur Bogenlinie nach Außen hin aufgefächert sind. Kurz gesagt sind die Führungsschlitze so zueinander verdreht und versetzt angeordnet, dass sich bei einer Verbindung der Flächenschwerpunkte einer Schnittfläche eine Bogenlinie abzeichnet. Dabei ergibt sich die Schnittfläche aus zwei sich überschneidenden Flächen. Die erste Fläche wird durch die Führungsschlitzöffnung gebildet, während die zweite Fläche durch den über den jeweiligen Führungsschlitz erzeugten Schnitt gebildet wird, genauer gesagt den Querschnitt des Schnitts, der einen Teil der facettenförmigen Außenkontur erzeugt. Die Schnittmenge dieser beiden sich überschneidenden Flächen ergibt schließlich die obengenannte Schnittfläche.

Durch eine derartige Anordnung der Führungsschlitze wird der Bewegungsfreiraum des knochenschneidenden Werkzeugs beim Erzeugen der durchgehenden Außenkontur erhöht. Dies wird dadurch ermöglicht, dass die Führungsschlitze von der Bogenlinie gesehen nach Außen bis zum dortigen Rand der sagittalen Führungsplatte ausgeführt werden können. Dies hat den Vorteil, dass bei sich nicht überschneidenden Führungsschlitzen das zwischen den Führungsschlitzen vorhandene Knochengewebe vorzugsweise durch schräges Einführen des knochenschneidenden Werkzeugs durchtrennt werden kann.

In einer weiteren besonders bevorzugten Ausführungsform weist die Schneidführung weiterhin eine transversale Spaltplatte auf, die von der sagittalen Führungsplatte zu der Seite absteht, in der sich die Führungsschlitze der sagittalen Führungsplatte durch die sagittale Führungsplatte erstrecken. Anders ausgedrückt befindet sich die transversale Spaltplatte auf der Seite der sagittalen Führungsplatte, auf der das Werkzeug aus den Führungsschlitzen der sagittalen Führungsplatte austritt. Die transversale Spaltplatte bildet somit zusammen mit der sagittalen Führungsplatte eine Art Winkelanschlag. Im Falle eines Kniegelenks liegt die transversale Spaltplatte nach Ausrichtung der sagittalen Führungsplatte am Knochen im Wesentlichen in der Transversalebene des Körpers beziehungsweise parallel hierzu.

Eine derartig ausgeführte transversale Spaltplatte ermöglicht ein einfaches Anlegen und Positionieren der Schneidführung an dem Knochen, an dem die Außenkontur erzeugt werden soll. Dabei kann die transversale Spaltplatte so ausgeführt sein, dass sie in den Gelenksspalt des Gelenks eingeführt werden kann, bevor eine der Gelenke reseziert wird. Es ist jedoch genauso im Sinne der Erfindung, die transversale Spaltplatte erst in den Gelenksspalt einzuführen, nachdem die gegenüberliegende Gelenkfläche bereits zur Aufnahme einer Gelenkendoprothese bearbeitet wurde. In so einem Fall kann die Stärke der Spaltplatte höher gewählt werden.

In einer weiteren Ausführungsform befindet sich in der oben ausgeführten transversalen Spaltplatte mindestens ein weiterer Führungsschlitz, der so durch die transversale Spaltplatte hindurch verläuft, dass er die Führungsschlitze der sagittalen Führungsplatte ergänzt, dass heißt die Reihe an Führungsschlitzen fortsetzt. Dabei ist der Führungsschlitz in der transversalen Spaltplatte vorzugsweise so angeordnet, dass er die Reihe der sagittalen Führungsschlitze vorzugsweise nach außen hin ergänzt, so dass ein durch den transversalen Führungsschlitz geführtes knochenschneidendes Werkzeug mindestens eine der außen liegenden Endflächen der Außenkontur erzeugt.

Die Anordnung eines Führungsschlitzes in der transversalen Spaltplatte hat dabei vor allem den Vorteil, dass die Schneidführung durch diese Ausführungsform eine kompaktere Bauweise erhält. Mit dieser Ausführungsform wird die Schneidführung um einen Führungsspalt ergänzt, ohne dabei die Abmessungen der Schneidführung zu vergrößern. Die kompaktere Ausführungsmöglichkeit der Schneidführung trägt dazu bei, dass zum Anbringen der Gelenkendoprothese ein möglichst kleiner Zugang zu dem Gelenk eröffnet wird und die Operationstechnik weniger invasiv ist.

In einer weiteren bevorzugten Ausführungsform weist die Schneidführung zudem einen anterioren Führungskörper auf, durch den ein im Wesentlichen zur sagittalen Führungsplatte paralleler Führungsschlitz verläuft und/oder mindestens ein Führungsloch. Im Falle eines Kniegelenks befindet sich der anteriore Führungskörper nach Ausrichtung der sagittalen Führungsplatte am Knochen anterior in Bezug zur Tibiagleitfläche.

Ein derartig angeordneter Führungsschlitz und oder ein derartig angeordnetes Führungsloch ermöglichen, dass nicht nur die Erzeugung der für die Implantation notwendigen Außengeometrie zum Anbringen des Gelenksersatzes ermöglicht wird, sondern zusätzlich die Geometrie der Außenkontur angepasst werden kann. So können zum Beispiel Gelenkendoprothesen auf ihrer Innenkontur mindestens ein Stift und/oder einen Steg aufweisen, für die eine entsprechende Aussparung, vorzugsweise eine Bohrung beziehungsweise eine Nut, in der angestrebten Außenkontur des Knochens vorzusehen ist.

Durch Integration des anterioren Führungskörpers in die Schneidführung können somit weitere Anforderungen an die Außengeometrie der Kontaktfläche zwischen Knochen und Prothese erfüllt werden. Dadurch, dass sämtliche Führungsschlitze und Führungslöcher der Schneidführung relativ zueinander feststehend positioniert angeordnet sind und die Schneidführung nur einmal angelegt und ausgerichtet werden muss, kann die Schneidführung auch bei den oben genannten komplexen Geometrieanforderungen eine korrekte Ausrichtung der einzelnen Abschnitte der Außengeometrie auf vorteilhafte Weise sicherstellen.

In einer Ausführungsform der vorliegenden Erfindung weist die Schneidführung mindestens einen Ausrichtungsvorsprung auf, der vorzugsweise an der transversalen Spaltplatte angeordnet ist, und dessen Scheitelpunkt als Drehpunkt zum Ausrichten der Schneidführung dient. Zu diesem Zweck sind die zum Scheitelpunkt führenden Flächen in mindestens einer Raumrichtung geneigt. Somit kann der Ausrichtungsvorsprung vorzugsweise an der gegenüberliegenden Gelenkseite zur Anlage gebracht werden und zwar entweder an dem intakten Gelenk oder an dem bereits mit einer Aussparung zur Aufnahme einer Gelenkprothese vorbereiteten Gelenkabschnitt. Der Ausrichtungsvorsprung kann als vorstehende Kante oder punktuelle Erhebung ausgeführt sein. Die über den Ausrichtungsvorsprung anliegende Schneidführung lässt sich somit relativ zu dem zu resezierenden Knochen durch Rotieren und/oder Kippen, also ggf. durch Drehbewegungen um mehr als eine Achse, mittels des Ausrichtungsvorsprungs relativ zum Knochenende ausrichten.

Ein so ausgeführter Ausrichtungsvorsprung ermöglicht eine einfache und genaue intraoperative Ausrichtung des Implantats. Anders gesagt kann ein derartiger Ausrichtungsvorsprung für die Ausrichtung des Implantats ausreichend sein. Selbstverständlich können aber auch ergänzende oder andere Ausrichtungsvorrichtungen eingesetzt werden.

In einer besonders bevorzugten Ausführungsform der Erfindung weist die Führung mindestens zwei Kontaktstellen auf, wobei vorzugsweise mindestens eine der Kontaktstellen hervorsteht. Über diese Kontaktstellen ist es möglich, die Schneidführung an die zu resezierende Gelenkoberfläche anzulegen. Bevorzugt ist zudem eine der Kontaktstellen als ein hervorstehender länglicher Körper ausgebildet, der von der sagittalen Führungsplatte, der transversalen Spaltplatte und/oder der Innenseite des anterioren Führungskörpers hervorsteht. Die Innenseite des anterioren Führungskörpers befindet sich auf der Seite, an der sich auch die Werkzeugaustrittsseite der sagittalen Führungsplatte befindet.

Der Abstand zwischen den Führungsschlitzen und den Kontaktstellen definiert die Tiefe der Schnittlinie, d.h. wie tief unter der Gelenkoberfläche die Außenkontur erzeugt wird. Dabei ist dieser Abstand primär von der Materialstärke der zu implantierenden Gelenkendoprothese abhängig.

Derartig ausgeführte Kontaktstellen ermöglichen ein gezieltes und schnelles Anlegen der Schneidführung an der zu resezierenden Gelenkoberfläche. Gleichzeitig erfolgt durch dieses Anlegen bereits die Positionierung oder zumindest die Vorpositionierung der Schneidführung relativ zur Gelenkoberfläche. Ein weiterer Vorteil ergibt sich dadurch, dass die Kontaktstellen zur Absicherung dienen können, ob als Ersatz für das Gelenk bzw. den Gelenkabschnitt die korrekte Implantatgröße gewählt wurde. Anders gesagt kann somit kontrolliert werden, dass dann eine korrekte Implantatgröße gewählt wurde, wenn die Kontaktstellen beim Anlegen der Schneidführung an die Gelenkoberfläche an den dafür vorgesehenen Stellen an dem Gelenk anliegen. Ist dies nicht der Fall, kann trotz Operationsplanung intraoperativ auch noch kurzfristig eine andere Implantatgröße über das Anlegen der dazugehörigen Schneidführung ausgewählt werden. Somit tragen die Kontaktstellen nicht nur zu einer optimalen Ausrichtung der Schneidführung bei, sondern ebenso zu einer korrekten Auswahl des anzubringenden Gelenksersatzes.

In einer weiteren Ausführungsform der vorliegenden Erfindung stehen die oben ausgeführten Kontaktstellen aus der sagittalen Führungsplatte, der transversalen Spaltplatte und/oder dem anterioren Führungskörper hervor. Dabei ist vorzugsweise an zumindest einer Kontaktstelle eine Markierung als weitere Positionierungshilfe für die Schneidführung vorhanden.

Insbesondere eine hervorstehende Kontaktstelle, wie beispielsweise ein aus der Schneidführung hervorstehender länglicher Körper, z.B. ein mit einer Markierung versehener Stift, hat dabei zum einen die Vorteile der Kontaktstellen der zuvor beschriebenen Ausführungsform, die zusätzliche Markierung ermöglicht jedoch außerdem die zu erzeugende Außenkontur in Richtung der sagittalen Führungsschlitze zu positionieren. Dies ist insbesondere dann wichtig, wenn die Gelenkendoprothese Führungsstifte oder Führungsfinnen aufweist, die in die zu erzeugende Außenkontur eingreifen. Hierfür müssen im Allgemeinen Aussparungen erzeugt werden, so dass die Schneidführung zuvor relativ zu der zu erzeugenden Außenkontur positioniert werden muss.

In einer weiteren Ausführungsform weist die Schneidführung mindestens ein Durchgangsloch, bevorzugt drei Durchgangslöcher, zur Befestigung der Schneidführung auf, wobei das Durchgangsloch vorzugsweise in der sagittalen Führungsplatte auf der Innenseite der Bogenlinie angeordnet ist. Über diese Durchgangslöcher wird die Schneidführung an dem Knochen der zu resezierenden Gelenkoberfläche befestigt. Die auf der Innenseite der Bogenlinie angeordneten Durchgangslöcher stellen dabei sicher, dass die Schneidführung auch nach dem Abtrennen von Gelenk- und Knochenteilen zum Erzeugen der Außenkontur sicher relativ zum Knochen befestigt bleibt. Die Befestigung der Schneidführung über das mindestens eine Durchgangsloch findet dabei vorzugsweise über Kirschnerdrähte, Klammern, Schrauben und/oder Nägel statt.

Weiterhin umfasst die Erfindung ein Verfahren zum Erzeugen einer Außenkontur an einem Knochen zum Anbringen einer Gelenkendoprothese, insbesondere einer Femurkomponente einer Knieendoprothese. Dabei wird zum Erzeugen der Außenkontur die zuvor beschriebene Schneidführung verwendet. Das Verfahren weist dabei die folgenden Schritte auf:
- Anlegen und Positionieren der Schneidführung an der lateralen und/oder medialen Seite des zu resezierenden Gelenks, Ausrichten der Schneidführung relativ zur Gelenkoberfläche,
- Erzeugen der Außenkontur mit einem knochenschneidenden Werkzeug und
- Abnehmen der Schneidführung.

Durch das Anlegen der Schneidführung an der lateralen und/oder medialen Seite des Knochens kann zunächst die korrekte Auswahl der Gelenkendoprothese durch Überprüfung einer korrekten Positionierung der Schneidführung relativ zur Gelenkoberfläche überprüft werden. Ist die Auswahl nicht korrekt, so kann die Schneidführung direkt wieder abgenommen werden und durch eine andere ersetzt werden. Andernfalls kann die Resektion der Gelenkoberfläche fortgesetzt werden. Ist die passende Schneidführung angelegt und ordnungsgemäß positioniert, kann mit der Resektion der Knochenoberfläche begonnen werden. Dabei ist als besonders vorteilhaft anzusehen, dass aufgrund der Anordnung der Werkzeugführungen an der Schneidführung die Resektion zur Erzeugung der gewünschten Außenkontur ohne Absetzen oder Umsetzen der Schneidführung erfolgt.

In einer weiteren besonders bevorzugten Ausführungsform des Verfahrens wird die Schneidführung über die mindestens zwei Kontaktstellen an die zu resezierende Gelenkoberfläche angelegt. Dabei ist bevorzugt mindestens eine der Kontaktstellen auf einer transversalen Spaltplatte angeordnet, die zuvor zwischen die beiden Gelenkseiten, bevorzugt zwischen mindestens einer Femurkondyle und dem Tibiaplateau, eingeschoben worden ist.

Nach Eröffnung des Zugangs zu dem Gelenk, wobei das Gelenk vorzugsweise in einer Flexions- oder Extensionsstellung gelagert wird, erleichtern die an der Schneidführung befindlichen Kontaktstellen das optimale Anlegen der Gelenkendoprothese an der zu resezierenden Gelenkoberfläche. Die Kontaktstellen erleichtern dabei außerdem eine Überprüfung, ob die richtige Prothesengröße ausgewählt wurde. Dies ist daran zu erkennen, ob die Kontaktstellen möglichst ohne Lichtspalt auf der Gelenkoberfläche aufliegen. Dabei werden als Kontaktstellen vorzugsweise besonders einfach zu identifizierende anatomische Orientierungspunkte gewählt. Befindet sich gemäß einer besonders bevorzugten Ausführungsform der Schneidführung weiterhin eine transversale Spaltplatte zwischen den beiden Gelenkseiten, so kann eine Überprüfung der Prothesengröße durch räumlich weiter auseinander liegende Kontaktstellen zusätzlich verbessert werden.

Sind insbesondere drei Kontaktstellen an der Schneidführung vorhanden, kann eine korrekte Wahl der Implantatgröße im Allgemeinen sofort erkannt werden, da in einem solchen Fall bevorzugt alle Kontaktstellen auf der Gelenkoberfläche zum Aufliegen kommen. Dabei liegt bevorzugt mindestens eine der Kontaktstellen an einer anatomisch einfach zu identifizierenden und inspizierenden Position an und dient somit als eine Art Referenzkontaktstelle.

In einer bevorzugten Ausführungsform des Verfahrens zur Resektion eines Kniegelenks liegen die Kontaktstellen der Schneidführung an mindestens einem der folgenden Bereiche der zu resezierenden Gelenkoberfläche an: dem Kontaktbereich, wo sich die gegenüberliegenden Gelenkflächen, bevorzugt die Femurkondyle und das Tibiaplateau, bei in etwa 85° Flexion berühren; dem Kontaktbereich, wo sich die gegenüberliegenden Gelenkflächen, bevorzugt die Femurkondyle und das Tibiaplateau, bei ungefähr 0° Extension berühren; und/oder dem Bereich, in dem ein Ende der zu implantierenden Gelenkendoprothese, vorzugsweise das anteriore Ende, an dem Knochen anliegt.

Insbesondere die hier beschriebenen anatomischen Orientierungspunkte lassen sich intraoperativ leicht identifizieren. Wird als eine der Kontaktstellen zudem eine Position gewählt, an der später eines der Enden der Gelenkendoprothese angrenzt, kann sichergestellt werden, dass die Prothese den gesamten Gleitbereich und somit den gesamten Bewegungsumfang des Gelenks überdeckt.

In einer bevorzugten Ausführungsform des Verfahrens wird vor dem Anlegen der Schneidführung eine Aussparung, vorzugsweise eine stufenförmige Aussparung, in die gegenüberliegende Gelenkseite eingebracht.

Das Einbringen einer bevorzugt stufenförmigen Aussparung in der gegenüberliegenden Gelenkseite erleichtert zusätzlich das Anlegen der Schneidführung an der zu resezierenden Gelenkoberfläche beziehungsweise speziell der transversalen Spaltplatte zwischen den beiden Gelenkseiten.

In einer weiteren bevorzugten Ausführungsform des Verfahrens wird die Schneidführung vor dem Erzeugen der Außenkontur über mindestens einen Ausrichtungsvorsprung, der als Drehpunkt für die Schneidführung dient, ausgerichtet.

Der an der Schneidführung vorhandene mindestens eine Ausrichtungsvorsprung stützt sich nach dem Anlegen und Positionieren der Schneidführung in dem Gelenk an der Außenkontur der gegenüberliegenden Gelenkseite ab. So lässt sich auf eine einfache Weise eine korrekte Ausrichtung der Gelenkendoprothese sicherstellen. Weiterhin ermöglichen die Ausrichtungsvorsprünge die Korrektur einer Varus/Valgus-Fehlstellung sowie eine Koordination des Zusammenspiels bei funktionell zusammengehörigen aber getrennten Gelenklaufflächen (z. B. Kniegelenk, Handgelenk oder Ellenbogengelenk).

Eine Kombination des mindestens einen Ausrichtungsvorsprungs und der Aussparung der vorstehenden Ausführungsform verbessert die Ausrichtung in der mindestens einen Raumrichtung, insbesondere aber auch eine bei zwei Ausrichtungsvorsprüngen mögliche Ausrichtung in zwei Raumrichtungen.

In einer besonders bevorzugten Ausführungsform des Verfahrens wird die Schneidführung nach dem Anlegen, Positionieren und Ausrichten über Durchgangslöcher an dem Knochen des zu resezierenden Gelenks befestigt.

Die Befestigung der Schneidführung nach dem Anlegen, Positionieren und Ausrichten stellt sicher, dass während der Erzeugung der Außenkontur am Knochen die Schneidführung ihre Position relativ zum Knochen beibehält. Natürlich gilt dies ebenso für das Erzeugen eines möglicherweise vorhandenen Führungslochs oder einer zusätzlichen Nut in der erzeugten oder noch zu erzeugenden Außengeometrie durch die Resektion der Gelenkoberfläche. Die Befestigung der Schneidführung erfolgt dabei beispielsweise mit Kirschnerdrähten, Klammern, Schrauben und/oder Nägeln, die eine besonders schnelle und effiziente Befestigung der Schneidführung ermöglichen.

In einer weiteren bevorzugten Ausführungsform des Verfahrens wird ein knochenschneidendes Werkzeug jeweils in die Führungsschlitze eingeführt, um das Gelenk zu resezieren und gleichzeitig über die hierbei ausgeführten Schnitte die gewünschte Außenkontur zum Anbringen der Gelenkendoprothese zu erzeugen.

Ein besonderer Vorteil durch die Verwendung der erfindungsgemäßen Schneidführung ergibt sich dadurch, dass die Resektion der Gelenkoberfläche und die Erzeugung der gewünschten Außenkontur mit ein und demselben Schnitt erfolgt. Neben dem nur einmaligen Anlegen der Schneidführung verkürzt auch dies die für die Resektion der Gelenkoberfläche notwendige Operationszeit.

In einer weiteren besonders bevorzugten Ausführungsform des Verfahrens wird vor und/oder nach dem Erzeugen der Außenkontur zum Einsetzen der Prothese über das mindestens eine anteriore Führungsloch, d.h. über das im anterioren Führungskörper angeordnete Führungsloch, ein Loch zur Aufnahme eines an der Endoprothese befindlichen Vorsprungs und/oder über den anterioren Führungsschlitz, d.h. über den im anterioren Führungskörper angeordneten Führungsschlitz, eine Nut zur Aufnahme einer an der Endoprothese befindlichen Finne in den Knochen eingebracht.

Das Verwenden ergänzender Schneidführungen wird insbesondere durch diese Ausführungsform des Verfahrens ermöglicht, da neben der Außenkontur zusätzlich oben bereits beschriebene Aussparungen in der Außengeometrie erzeugt werden können. Diese ermöglichen die Aufnahme möglicherweise vorhandener Stifte oder Finnen an der Innenkontur der Endoprothese, die mit der Außenkontur nach der Implantation in Kontakt steht.

### KURZE BESCHREIBUNG DER FIGUREN

Im Folgenden wird die Erfindung anhand beispielhafter, in den beigefügten Figuren dargestellter Ausführungsformen erläutert. Es zeigt:
Figur 1 eine perspektivische Ansicht der anterioren Seite der Schneidführung,
Figur 2 eine weitere perspektivische Ansicht der anterioren Seite der Schneidführung,
Figur 3 eine Seitenansicht der anterioren Seite einer erfindungsgemäßen Schneidführung,
Figur 4 eine Draufsicht einer erfindungsgemäßen Schneidführung mit einem Drehpunkt zur Ausrichtung des Implantats in einer Raumrichtung,
Figur 5 das Anlegen einer erfindungsgemäßen Schneidführung an einer Gelenkseite,
Figur 6 das Anlegen der Schneidführung in einer Draufsicht und einer anterioren Seitenansicht an einem Kniegelenk,
Figur 7 eine Seitenansicht einer implantierten Gelenkendoprothese, das heißt in diesem Fall eines Gelenkoberflächenersatzes, am Beispiel eines Knies, und
Figur 8 eine Seitenansicht einer erfindungsgemäßen Schneidführung aus der Sicht des zu resezierenden Gelenks.
Figur 9 ist eine schematische Ansicht zur Anordnung der Führungsschlitze in der sagittalen Führungsplatte.

### AUSFÜHRLICHE BESCHREIBUNG BEVORZUGTER AUSFÜHRUNGSFORMEN

Im Folgenden werden bevorzugte Ausführungsformen der vorliegenden Erfindung anhand der zugehörigen Figuren erläutert. Gleiche Bezugszeichen bezeichnen soweit nicht anders ausgeführt im Allgemeinen gleiche oder gleichwirkende Merkmale der vorliegenden Erfindung.

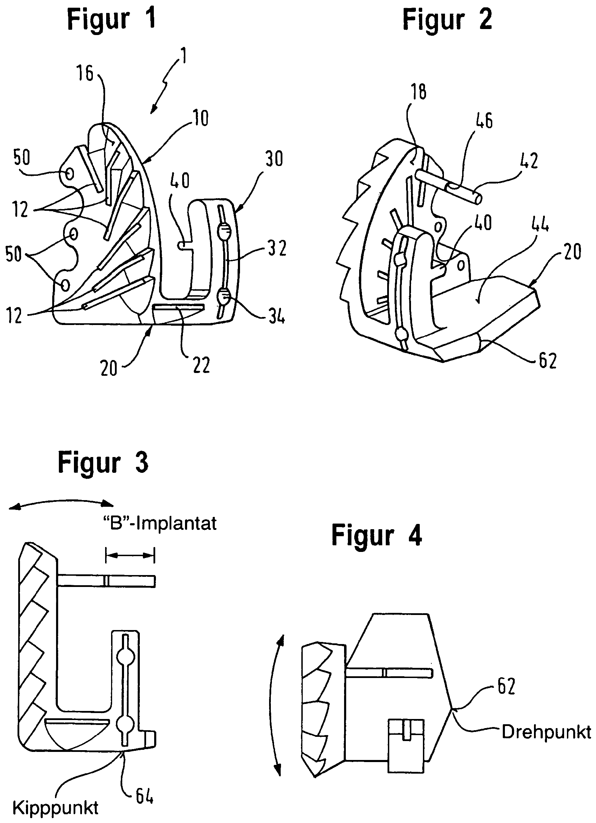

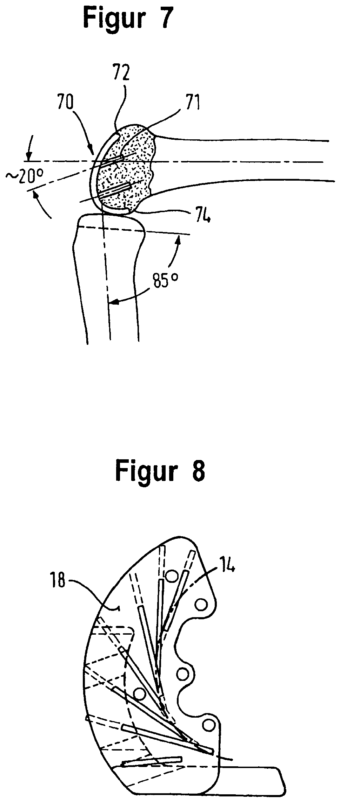

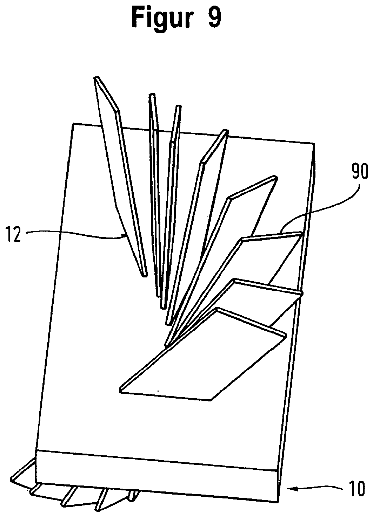

Figur 1 zeigt eine erfindungsgemäße Ausführungsform der Schneidführung 1. Dabei sind sowohl die sagittale Führungsplatte 10 wie auch das anteriore Ende der transversalen Spaltplatte 20 und der anteriore Führungskörper 30 dargestellt. Die sagittale Führungsplatte weist wie in den Figuren 2 und 8 gezeigt eine sagittal verlaufende Seitenfläche 18 auf. Die aus der Seitenfläche 18 austretenden Führungsschlitze 12 sind auf einer in Figur 8 dargestellten Bogenlinie 14 aufgereiht. Dabei sind die sagittalen Führungsschlitze 12 sowohl zueinander verdreht als auch zueinander versetzt angeordnet und zwar so, dass sie aus Sicht der Bogenlinie nach außen auffächern. Die in Figur 1 gezeigten Führungsschlitze 12, 22 und 32 sind als Führungsschlitze für eine Säge ausgebildet, insbesondere eine Oszillationssäge. Dies wird beispielhaft in Figur 9 gezeigt. Dabei kennzeichnet das Bezugszeichen 90 ein schematisch angedeutetes Oszillationssägeblatt, welches in die Führungsschlitze 12 eingeführt wird. Zur Verdeutlichung der winkligen geneigten Anordnung der einzelnen Führungsschlitze zueinander, befindet sich in jedem Führungsschlitze 12 ein Sägeblatt 90. Jedes dieser Sägeblätter ist um eine im Wesentlichen parallel zur Schlitzbreite verlaufenden Achse geneigt. Die Führungsschlitze verlaufen auf der sagittalen Führungsplatte tangential zu einer imaginären Bogenlinie und stehen winklig voneinander ab. Wie oben beschrieben, ist es allerdings genauso möglich, die Führungsschlitze für ein knochentrennendes Werkzeug wie eine Fräse auszuführen.

Die Führungsschlitze können wie in Figur 1 bei den unteren sagittalen Führungsschlitzen 12 dargestellt als in sich geschlossene Schlitze ausgeführt sein. Genauso ist es allerdings möglich, sie zur Außenseite der sagittalen Platte hin offen auszuführen, wie beispielsweise bei dem sagittalen Führungsschlitz im oberen Bereich der Figuren 1 und 2 gezeigt. Hinzu kommt, dass die Führungsschlitze keinesfalls die gleiche Länge aufweisen müssen, sondern durchaus auch verschieden lang ausgeführt werden können.

Weiterhin zeigen die Figuren 1 und 2, dass die sagittalen Führungsschlitze 12 zwar winklig zueinander angeordnet sind, jedoch nicht immer um den gleichen Winkel zueinander verdreht sind. Weiterhin sind jeweils an den äußeren Enden der sagittalen Führungsschlitze 12 Einführflächen 16 für das knochenschneidende Werkzeug vorgesehen, die das Einführen des Werkzeugs in die Führungsschlitze erleichtern und zudem vorzugsweise einseitig die zur Verfügung stehende Führungsfläche erhöhen. In einer bevorzugten Ausführungsform der Erfindung dienen die Enden der Einführflächen 16 dabei als Orientierungshilfe beim Einführen des Werkzeugs in die Führungsschlitze. So können die Enden der Einführflächen 16 bevorzugt so ausgestaltet sein, dass die Längsachse des Werkzeugs, z.B. der Oszillationssäge, beim Einführen in einen Führungsschlitz vorzugsweise im rechten Winkel zur Abschlusskante der Einführfläche 16 steht. Dies gewährleistet ebenfalls eine kurze Operationszeit sowie eine reproduzierbar hohe Qualität beim Erzeugen der Außenkontur an einem Knochen.

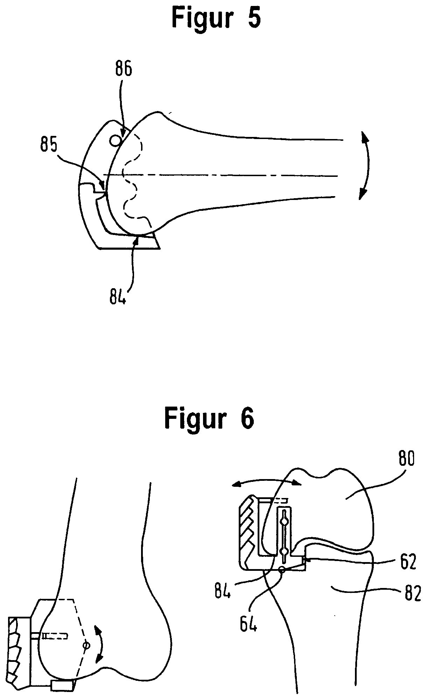

Auf der Innenseite der Bogenlinie 14 sind in Figur 1 drei Durchgangslöcher 50 angeordnet, über die die Schneidführung 1 an dem zu resezierenden Gelenk 80 (siehe Figur 6) befestigt ist. Vorzugsweise sind die Durchgangslöcher 50 alle in einem Bereich angeordnet, der bei der Befestigung der Schneidführung 1 nicht in den Knochenbereich der Resektion fällt. Auch wenn die Befestigungslöcher 50 in dem gezeigten Ausführungsbeispiel in derselben Raumrichtung verlaufen wie die sagittalen Führungsschlitze 12, ist es ebenso möglich sie in einer anderen Raumrichtung auszurichten oder auch an einem anderen Abschnitt der Schneidführung 1 vorzusehen.

Wie in den Figuren 1 bis 6 dargestellt, ist eine transversale Spaltplatte 20 senkrecht zu der sagittalen Führungsplatte 10 angeordnet. Selbstverständlich ist es möglich, die transversale Spaltplatte 20 auch in einem anderen Winkel anzuordnen. Entscheidend ist vor allem die Ausrichtung und Positionierung der Führungselemente zueinander, d.h. der Führungsschlitze 12, 22, 32 sowie der Führungslöcher 34. Wie in Figur 1 gezeigt, ist der anteriore Führungsschlitz 22 im Verhältnis zu den sagittalen Führungsschlitzen 12 so angeordnet, dass er die Reihe der sagittalen Führungsschlitze 12 im unteren Bereich wirkungstechnisch um einen weiteren Führungsschlitz ergänzt. In der gezeigten beispielhaften Ausführungsform bildet der Führungsschlitz 22 die Werkzeugführung für die Erzeugung einer am äußeren Ende liegenden Facettenfläche der Außenkontur.

Die transversale Spaltplatte 20 ist wie in den Figuren 1, 2, 4, 5 und 6 gezeigt bevorzugt so ausgeführt, dass sie über die sagittale Führungsplatte 10 und den anterioren Führungskörper 30 hinausragt. Damit wird sichergestellt, dass sie möglichst weit in den Zwischenraum der gegenüberstehenden Gelenkseiten 80, 82 eingeführt werden kann. Zudem weist die transversale Spaltplatte 20 eine Kontaktstelle 44 auf, bei dem beispielhaft in Figur 5 der durch seine Lage anatomisch gut zu identifizierende Orientierungspunkt 84 anliegt.

Die Figuren 2 und 4 zeigen einen Drehpunkt 62, der in diesem erfindungsgemäßen Ausführungsbeispiel als vorstehende Kante ausgeführt ist. Auf der der Kontaktstelle 44 der transversalen Spaltplatte 20 gegenüberliegenden Seite befindet sich ein Kipppunkt 64, der in dem dargestellten Ausführungsbeispiel in Figur 3 nicht als Kante, sondern als ein aus der transversalen Spaltplatte 20 hervorstehender punktförmiger Vorsprung ausgebildet ist.

Anhand der Figur 6 wird die Funktionsweise der Ausrichtungsvorsprünge, d. h. sowohl des Drehpunktes 62 als auch des Kipppunktes 64 deutlich. Der Ausrichtungsvorsprung des Drehpunkts 62 und/oder des Kipppunkts 64 kann als vorstehende Kante oder als vorstehende Punktauflage ausgebildet sein. Dabei ermöglicht der Kipppunkt 64 bei der Ausrichtung der Schneidführung 1 beispielsweise auch, wenn notwendig, eine Korrektur einer Varus-Valgus-Fehlstellung durch entsprechendes Kippen der Schneidführung 1 vor dem Erzeugen der Außenkontur des Knochens. Dabei ist es neben der Auflage auf dem Gelenkknorpel auch wie in Figur 6 gezeigt möglich, die Ausrichtung über den Kipppunkt 64 bei Aufliegen der Schneidführung 1 auf einer bereits operativ erzeugten Aussparung in der gegenüberliegenden Gelenkseite auszuführen. Dasselbe gilt für den Drehpunkt 62, der in der in Figur 6 gezeigten Ausführungsform zusätzlich eine Ausrichtung der Schneidführung 1 um eine zweite Rotationsachse zulässt. Bei Verwendung der Schneidführung 1 zur Resektion einer Femurkondyle ermöglicht dies eine Ausrichtung der Schneidführung und damit der anzubringenden Gelenkendoprothese in der Rotationsrichtung um die Längsachse des Femurs.

Weiterhin ist in den Figuren 1 bis 6 der anteriore Führungskörper 30 als weiterer Abschnitt der erfindungsgemäßen Schneidführung 1 ausgebildet. Der hier ausgeführte anteriore Führungskörper 30 weist dabei eine Kontaktstelle 40, zwei Führungslöcher 34 und einen Führungsschlitz 32 auf. Hierbei ist anzumerken, dass der anteriore Führungskörper 30 im Allgemeinen mindestens eines dieser Merkmale aufweist, es aber keinesfalls zwingend erforderlich ist, dass alle diese Merkmale vorhanden sind. Sowohl der Führungsschlitz 32 wie auch die Führungslöcher 34 sind im Verhältnis zu den sagittalen Führungsschlitzen 12 senkrecht zueinander ausgerichtet. Es ist jedoch durchaus möglich, einen davon abweichenden Winkel, vorzugsweise zwischen 60 und 120° zu wählen, beispielsweise, wenn ein solcher Winkel für eine Anpassung der Außenkontur des Knochens an die Innenkontur der Gelenkendoprothese erforderlich ist. Auf der Werkzeugausgangsseite der Führungslöcher 34 beziehungsweise des Führungsschlitzes 32 befindet sich eine als Vorsprung ausgeführte Kontaktstelle 40. Dabei ist die Kontaktstelle 40 als Anlegekante ausgeführt. Sie kann jedoch genauso als Fläche ausgeführt sein, wie zum Beispiel bei der Kontaktstelle 44 der transversalen Spaltplatte 20 der Fall oder auch als Anlagepunkt (nicht gezeigt).

Die flächig ausgeführte Kontaktstelle 44 der transversalen Spaltplatte 20 liegt in Bezug zur Gelenkfläche des zu resezierenden Gelenks 80 an dem anatomisch gut identifizierbaren Orientierungspunkt 84. In ähnlicher Weise liegt die Kontaktstelle 40 des anterioren Führungskörpers 30 an dem anatomischen Orientierungspunkt 85 an. In dem gezeigten Beispiel handelt es sich bei dem anatomischen Orientierungspunkt 84 um den Kontaktbereich zwischen den Gelenkflächen des Kniegelenks bei 85° Flexion und bei dem Orientierungspunkt 85 um den Kontaktbereich zwischen Femur und Tibiaplateau bei 0° Extension.

Mit den Führungslöchern 34 werden vorzugsweise mit einem Bohrer Löcher in die zu erzeugende Außenkontur eingebracht, so dass an der Gelenkendoprothese vorhandene Stifte 71 von der erzeugten Knochenaußenkontur des zu resezierenden Gelenks aufgenommen werden können. Wie in Figur 7 gezeigt, sind die Führungslöcher 34 so in dem anterioren Führungskörper 30 ausgerichtet, dass mit ihnen Löcher in der zu erzeugenden Außenkontur erzeugt werden können, die vorzugsweise ungefähr 20° zur Längsachse des Femurs des Kniegelenks ausgerichtet sind.

Eine zu den Führungslöchern 34 ähnliche Funktion erfüllt der Führungsschlitz 32 indem ein in ihn eingeführtes knochentrennendes Werkzeug, wie zum Beispiel eine Säge oder eine Fräse, eine zusätzliche Nut in die zu erzeugende Außenkontur des Knochens einarbeitet. Über eine entsprechend ausgebildete Nut ist es möglich, eine Gelenkendoprothese mit einer Finne zu implantierten.

In den Figuren 2 bis 6 ist weiterhin gezeigt, dass mindestens eine Kontaktstelle auch als länglicher vorstehender Körper ausgeführt werden kann. So ist in der dort vorliegenden Ausführungsform ein zylindrisch ausgeführter Stift mit Kontaktstelle 42 im Wesentlichen senkrecht zu der sagittalen Führungsplatte 10 angeordnet. Die in den Figuren 2 bis 4 sowie Figur 6 gezeigte Kontaktstelle 42 weist weiterhin eine Markierung 46 auf, die entlang ihrer Längsrichtung angebracht ist.

Zwar kann die Kontaktstelle 42 ebenso an einem anatomischen Orientierungspunkt des zu resezierenden Gelenks anliegen, jedoch ist es wie in Figur 5 gezeigt genauso möglich, dass die Anlegestelle 42 in einem Bereich an dem Gelenk anliegt, wo sich nach der Implantation eines der Enden der Gelenkendoprothese befindet.

Wie in den Figuren 5 und 6 gezeigt, dient die Markierung 46 dazu, die Schneidführung 1 in einem festgelegten Abstand zum Gelenk 80 zu positionieren. Dabei wird die Schneidführung 1 soweit an das Gelenk 80 angenähert, bis die Markierung 46 und die Außenkontur des zu resezierenden Gelenks übereinander liegen. Als Alternative oder Ergänzung zu der in den Figuren dargestellten Ausführungsformen kann die Markierung auch an einer beliebigen anderen Stelle der Schneidführung 1 bereitgestellt sein oder durch eine Kante, beispielsweise einer Kante des anterioren Führungskörpers 30, gebildet werden.

## Patentansprüche

1. Schneidführung (1) zum Erzeugen einer Außenkontur an einem Knochen, wobei an der Außenkontur eine Gelenkendoprothese (70) anbringbar ist; die Schneidführung weist eine sagittale Führungsplatte (10) auf, die zu dem Knochen ausrichtbar ist, und mindestens zwei sagittale Führungsschlitze (12) zum Führen eines knochenschneidenden Werkzeugs, wobei die sagittalen Führungsschlitze (12) durch die sagittale Führungsplatte (10) hindurchgehen und winklig zueinander angeordnet sind, wobei jeder sagittale Führungsschlitz (12) eine in sich abgeschlossene Öffnung bildet,
**dadurch gekennzeichnet, dass**
die Führungsschlitze derart angeordnet sind, dass die durch sie erzeugte Außenkontur am Knochenende durch facettenartig angeordnete, aneinander angrenzende Flächen ausgebildet wird.

2. Schneidführung (1) nach Anspruch 1, **dadurch gekennzeichnet, dass**
die Führungsschlitze (12) auf der sagittalen Führungsplatte (10) im Wesentlichen auf einer Bogenlinie (14) angeordnet sind und zwar vorzugsweise so, dass sie mit ihrer Längsrichtung winklig zu der Bogenlinie (14) ausgerichtet und relativ zur Bogenlinie (14) nach außen hin aufgefächert sind.

3. Schneidführung (1) nach einem der vorstehenden Ansprüche, **dadurch gekennzeichnet, dass** sie weiterhin eine transversale Spaltplatte (20) aufweist, die von der sagittalen Führungsplatte (10) in der Richtung absteht, in der sich die Führungsschlitze (12) der sagittalen Führungsplatte (10) durch die sagittale Führungsplatte erstrecken.

4. Schneidführung (1) nach Anspruch 3, **dadurch gekennzeichnet, dass**
sich in der transversalen Spaltplatte (20) mindestens ein weiterer Führungsschlitz (22) befindet, der so durch die transversale Spaltplatte (20) hindurch verläuft, dass er die Führungsschlitze (12) der sagittalen Führungsplatte (10) ergänzt.

5. Schneidführung (1) nach einem der vorstehenden Ansprüche, **dadurch gekennzeichnet, dass**
sie weiterhin einen anterioren Führungskörper (30), durch den ein im Wesentlichen zur sagittalen Führungsplatte (10) paralleler Führungsschlitz (32) verläuft und/oder mindestens ein Führungsloch (34) aufweist.

6. Schneidführung (1) nach einem der vorstehenden Ansprüche, **dadurch gekennzeichnet, dass**
sie mindestens einen Ausrichtungsvorsprung (62, 64) aufweist, der vorzugsweise an der transversalen Spaltplatte (20) angeordnet ist, und der als Drehpunkt zum Ausrichten der Schneidführung in mindestens einer Raumrichtung geeignet ist.

7. Schneidführung (1) nach einem der vorstehenden Ansprüche, **dadurch gekennzeichnet, dass**
die Schneidführung (1) mindestens zwei Kontaktstellen (40, 42, 44) aufweist, über die die Schneidführung (1) angelegt werden kann, wobei mindestens eine der Kontaktstellen hervorsteht.

8. Schneidführung (1) nach Anspruch 7, **dadurch gekennzeichnet, dass** die hervorstehende Kontaktstelle (40, 42, 44) von der sagittalen Führungsplatte (10), der transversalen Spaltplatte (20) und/oder dem anterioren Führungskörper (30) hervorsteht und an zumindest einer der Kontaktstellen eine Markierung (46) als weitere Positionierungshilfe für die Schneidführung (1) vorhanden ist.

9. Schneidführung (1) nach einem der vorstehenden Ansprüche, **dadurch gekennzeichnet, dass** sie mindestens ein Durchgangsloch (50) zur Befestigung aufweist, das vorzugsweise in der sagittalen Führungsplatte (10) relativ zu der Bogenlinie (14) nach innen angeordnet ist.

10. Schneidführungsset zum Erzeugen einer Außenkontur an einem Kniegelenk, umfassend zwei laterale und mediale Schneidführungen gemäß einem der vorstehenden Ansprüche für mindestens eine Gelenkendoprothesengröße, die jeweils spiegelverkehrt ausgeführt sind.

## Claims

1. Cutting guide (1) for generating an outer contour on a bone, wherein a joint endoprosthesis (70) can be applied to the outer contour; the cutting guide has a sagittal guide plate (10), which can be oriented with respect to the bone, and at least two sagittal guide slits (12) for guiding a bone-cutting tool, wherein the sagittal guide slits (12) pass through the sagittal guide plate (10) and are arranged at an angle to each other, wherein each sagittal guide slit (12) forms a self-contained opening, **characterized in that** the guide slits are arranged in such a way that the outer contour generated by them at the bone end is formed by mutually adjoining surfaces arranged in the manner of facets.

2. Cutting guide (1) according to Claim 1, **characterized in that** the guide slits (12) on the sagittal guide plate (10) are arranged substantially on an arc line (14), indeed preferably such that they are oriented with their longitudinal direction at an angle to the arc line (14) and fan out towards the outside relative to the arc line (14).

3. Cutting guide (1) according to one of the preceding claims, **characterized in that** it moreover has a transverse cleft plate (20), which protrudes from the sagittal guide plate (10) in the direction in which the guide slits (12) of the sagittal guide plate (10) extend through the sagittal guide plate.

4. Cutting guide (1) according to Claim 3, **characterized in that**, in the transverse cleft plate (20), there is at least one further guide slit (22), which extends through the transverse cleft plate (20) such that it supplements the guide slits (12) of the sagittal guide plate (10).

5. Cutting guide (1) according to one of the preceding claims, **characterized in that** it moreover has an anterior guide body (30) through which a guide slit (32) that is substantially parallel to the sagittal guide plate (10) extends and/or at least one guide hole (34).

6. Cutting guide (1) according to one of the preceding claims, **characterized in that** it has at least one orientation projection (62, 64), which is preferably arranged on the transverse cleft plate (20) and which is suitable as a rotation point for the orientation of the cutting guide in at least one spatial direction.

7. Cutting guide (1) according to one of the preceding claims, **characterized in that** the cutting guide (1) has at least two contact points (40, 42, 44) via which the cutting guide (1) can be put in place, wherein at least one of the contact points is protruding.

8. Cutting guide (1) according to Claim 7, **characterized in that** the protruding contact point (40, 42, 44) protrudes from the sagittal guide plate (10), the transverse cleft plate (20) and/or the anterior guide body (30), and a marking (46) serving as a further positioning aid for the cutting guide (1) is present on at least one of the contact points.

9. Cutting guide (1) according to one of the preceding claims, **characterized in that** it has at least one through-hole (50) for fastening purposes, which is preferably arranged in the sagittal guide plate (10) towards the inside relative to the arc line (14).

10. Cutting guide set for generating an outer contour on a knee joint, comprising two lateral and medial cutting guides according to one of the preceding claims for at least one joint endoprosthesis size, which cutting guides each have a mirror-inverted design.

## Revendications

1. Guidage de coupe (1) pour produire un contour externe sur un os, dans lequel une endoprothèse articulée (70) peut être posée sur le contour extérieur; le guidage de coupe présente une plaque de guidage sagittale (10), qui peut être orientée vers l'os, et au moins deux fentes de guidage sagittales (12) pour le guidage d'un outil de coupe de l'os, dans lequel les fentes de guidage sagittales (12) percent la plaque de guidage sagittale (10) et sont disposées avec un angle l'une par rapport à l'autre, dans lequel chaque fente de guidage sagittale (12) forme une ouverture par nature fermée, **caractérisé en ce que** les fentes de guidage sont disposées de telle manière que le contour extérieur produit par celles-ci sur l'extrémité de l'os soit réalisé par des faces adjacentes l'une à l'autre disposées à la manière de facettes.

2. Guidage de coupe (1) selon la revendication 1, **caractérisé en ce que** les fentes de guidage (12) sont disposées sur la plaque de guidage sagittale (10) essentiellement sur une ligne arquée (14), notamment de préférence de telle manière qu'elles soient orientées avec leur direction longitudinale faisant un angle avec la ligne arquée (14) et étalées en éventail vers l'extérieur par rapport à la ligne arquée (14).

3. Guidage de coupe (1) selon l'une quelconque des revendications précédentes, **caractérisé en ce qu'**il présente en outre une plaque fendue transversale (20), qui est saillante sur la plaque de guidage sagittale (10) dans la direction dans laquelle les fentes de guidage (12) de la plaque de guidage sagittale (10) s'étendent à travers la plaque de guidage sagittale.

4. Guidage de coupe (1) selon la revendication 3, **caractérisé en ce qu'**il se trouve dans la plaque fendue transversale (20) au moins une autre fente de guidage (22), qui s'étend à travers la plaque fendue transversale (20) de telle manière qu'elle complète les fentes de guidage (12) de la plaque de guidage sagittale (10).

5. Guidage de coupe (1) selon l'une quelconque des revendications précédentes, **caractérisé en ce qu'**il présente en outre un corps de guidage antérieur (30), à travers lequel s'étend une fente de guidage (32) essentiellement parallèle à la plaque de guidage sagittale (10), et/ou au moins un trou de guidage (34).

6. Guidage de coupe (1) selon l'une quelconque des revendications précédentes, **caractérisé en ce qu'**il présente au moins une saillie d'orientation (62, 64), qui est de préférence disposée sur la plaque fendue transversale (20), et qui convient comme point de rotation pour l'orientation du guidage de coupe dans au moins une direction de l'espace.

7. Guidage de coupe (1) selon l'une quelconque des revendications précédentes, **caractérisé en ce que** le guidage de coupe (1) présente au moins deux points de contact (40, 42, 44), par lesquels le guidage de coupe (1) peut être posé, dans lequel au moins un des points de contact est saillant.

8. Guidage de coupe (1) selon la revendication 7, **caractérisé en ce que** le point de contact saillant (40, 42, 44) est saillant sur la plaque de guidage sagittale (10), sur la plaque fendue transversale (20) et/ou sur le corps de guidage antérieur (30), et il se trouve sur au moins un des points de contact un marquage (46) à titre d'aide supplémentaire au positionnement pour le guidage de coupe (1).

9. Guidage de coupe (1) selon l'une quelconque des revendications précédentes, **caractérisé en ce qu'**il présente au moins un trou traversant (50) pour la fixation, qui est disposé de préférence dans la plaque de guidage sagittale (10) vers l'intérieur par rapport à la ligne arquée (14).

10. Ensemble de guidage de coupe pour produire un contour extérieur sur une articulation du genou, comprenant deux guides de coupe latéraux et médiaux selon l'une quelconque des revendications précédentes, pour au moins une taille d'endoprothèse articulée, qui sont respectivement réalisés de façon symétriquement inverse.
